# EUROPÄISCHE PATENTSCHRIFT

(11) **EP 2 018 043 B1**
(45) Veröffentlichungstag und Bekanntmachung des Hinweises auf die Patenterteilung: **03.09.2014**
(21) Anmeldenummer: 08011821.9
(22) Anmeldetag: 01.07.2008
(51) Int. Cl.: H04N 5/225

(54) **Bildaufnehmermodul**
Imaging module
Module imageur

(30) Priorität: 18.07.2007 DE 102007034704
(43) Veröffentlichungstag der Anmeldung: 21.01.2009
(73) Patentinhaber: Karl Storz GmbH & Co. KG, 78532 Tuttlingen (DE)
(72) Erfinder: Schwarz, Peter, 78532 Tuttlingen (DE); Graf, Christian, 78576 Emmingen-Liptingen (DE); Irion, Klaus-Martin, Dr.-Ing., 78576 Emmingen-Liptingen (DE)
(74) Vertreter: Heuckeroth, Volker

(56) Entgegenhaltungen:
- DE-A1-102004 056 946
- US-A1- 2002 080 233
- US-A1- 2007 158 773

## Beschreibung

Die Erfindung betrifft ein Bildaufnehmermodul, insbesondere für ein Endoskop oder eine Miniaturkamera, mit einem elektronischen Bildsensor, der eine Mehrzahl von Kontaktfingern aufweist, die in zumindest einer Reihe angeordnet sind, und mit einer starren Platine, mit der die Kontaktfinger elektrisch kontaktiert sind, wobei der Bildsensor und die Platine etwa parallel zueinander angeordnet sind, und wobei die Kontaktfinger sich entlang zumindest einer, sich etwa quer zum Bildsensor erstreckenden Längsseite der Platine erstrecken, ferner mit einem flexiblen mehradrigen Kabel, das von der Platine in Richtung von dem Bildsensor wegführt und dessen Adern ebenfalls mit der Platine elektrisch kontaktiert sind, wobei die Adern an Kontaktierstellen an der Platine kontaktiert sind, die näher zum Bildsensor liegen als die vom Bildsensor abgewandte Seite der Platine.

Ein solches Bildaufnehmermodul ist aus dem Dokument JP 2001 178675 A bekannt.

Ohne Beschränkung der Allgemeinheit wird ein derartiges elektronisches Bildaufnehmermodul in einem Endoskop, insbesondere in einem flexiblen Endoskop verwendet, wobei das Bildaufnehmermodul im distalen Ende des Endoskopschafts angeordnet wird. Ein solches Endoskop bzw. Videoendoskop ist beispielsweise aus US 5,754,313 bekannt.

Ein Bildaufnehmermodul umfasst allgemein einen elektronischen Bildsensor bzw. Bildaufnehmer, der auf ihn einfallendes Licht in ein elektrisches Signal umwandelt. Allgemein sind solche elektronischen Bildsensoren in CCD- oder CMOS-Technologie ausgeführt.

Miniaturisierte Bildsensoren sind derzeit erhältlich, unter denen solche bevorzugt werden, die in TAB(Tape Automated Bonding)-Technologie gefertigt sind. Derartige Bildsensoren weisen in zumindest einer Reihe, üblicherweise in zwei Reihen an gegenüberliegenden Seiten des Bildsensors angeordnete Kontaktfinger auf, die sich etwa senkrecht zur Lichteintrittsfläche des Bildsensors von diesem weg erstrecken und mit der Platine des Bildaufnehmermoduls kontaktiert sind. Die Kontaktfinger eines Bildsensors, der in TAB-Form gefertigt wird, liegen in der gleichen Ebene wie der Bildsensor. Die Kontaktfinger werden umgebogen und stehen erst nach dem Umbiegen etwa senkrecht zur Lichteintrittsfläche des Bildsensors.

Unter "Platine" werden im Sinne der vorliegenden Erfindung sowohl einstückige, einteilige als auch mehrstückige bzw. mehrteilige Platinen verstanden, wobei in bestimmten Ausgestaltungen bestimmte dieser Bauweisen bevorzugt sind, wie später noch beschrieben wird.

Die Platine (Leiterplatte) dient nicht nur zur Kontaktierung der Kontaktfinger des Bildsensors, sondern auch zur Aufnahme elektronischer Bauelemente, die für die Steuerelektronik und/oder Ansteuerung des Bildsensors benötigt werden.

Des Weiteren ist mit der Platine ein flexibles mehradriges Kabel kontaktiert, über das die vom Bildsensor erzeugten elektrischen Videosignale zur Kamerakontrolleinheit geführt bzw. Ansteuer- und Versorgungssignale zur Platine zugeführt werden.

Aus dem eingangs genannten Dokument JP 2001 178675 A ist ein Bildaufnehmermodul bekannt, das eine starre Platine aufweist, die im Abstand zu dem Bildsensor und parallel zu diesem angeordnet ist. Ein flexibles mehradriges Kabel ist mit der Platine elektrisch kontaktiert, wobei die einzelnen Adern mit ihren abisolierten Enden in Bohrungen in der Platine eingeführt und in diesen Bohrungen mit der Platine kontaktiert sind.

Ein damit vergleichbares Bildaufnehmermodul ist aus JP 2000 092477 A bekannt.

JP 2000 209472 A offenbart ein Bildaufnehmermodul, dessen starre Platine als Multilayerplatine ausgebildet ist. Die Adern des mehradrigen flexiblen Kabels sind an Außenseiten der Platine mit der Platine kontaktiert, wobei die Kontaktfinger des Bildsensors an den beiden anderen Außenseiten der Platine mit dieser kontaktiert sind.

US 2007/158773 A1 offenbart ein Bildaufnehmermodul, das einen Bildsensor mit Kontaktfingern aufweist, wobei der Bildsensor in ein Modulgehäuse herausnehmbar eingesteckt ist, wobei das Modulgehäuse Kontaktelemente aufweist, die beim Einstecken des Bildsensors in das Modulgehäuse mit den Kontaktfingern des Bildsensors elektrisch kontaktiert werden. Dieses Bildaufnehmermodul weist eine Platine auf, wobei in dem Dokument nicht offenbart ist, wie die Platine mit einem externen mehradrigen Kabel kontaktiert ist.

Die Platine eines aus dem Dokument DE 10 2004 056 946 A1 bekannten Bildaufnehmermoduls ist einstückig und starr ausgebildet, und besitzt die Form eines U, wobei die U-förmige Ausgestaltung durch Ausfräsen von Material aus einem würfel- oder quaderförmigen Platinenrohling hergestellt wird. Diese Platine ist entsprechend der U-förmigen Ausgestaltung an zwei gegenüberliegenden Längsseiten offen und an den verbleibenden zwei gegenüberliegenden Längsseiten geschlossen. "Längsrichtung" bezeichnet in der vorliegenden Beschreibung die Richtung senkrecht zur Lichteintrittsfläche des Bildsensors.

In der durch die U-förmige Gestalt gebildeten Nut der Platine des bekannten Bildaufnehmermoduls sind elektrische Bauelemente in dreidimensionaler Anordnung mit der Platine kontaktiert, d.h. mehrere elektronische Bauelemente sind übereinander gestapelt.

Das mehradrige flexible Kabel ist dabei auf der dem Bildsensor abgewandten Außenseite der Platine, die nachfolgend als "Unterseite" der Platine bezeichnet wird, mit dieser kontaktiert. Obwohl das bekannte Bildaufnehmermodul in Längsrichtung gewünschtermaßen sehr kurz baut, hat die Kontaktierung des mehradrigen Kabels an der dem Bildsensor abgewandten Außenseite der Platine den Nachteil, dass der starre Teil des insgesamt starren Bildaufnehmermoduls durch das kontaktierte Kabelassembly unerwünscht verlängert wird, weil die Kontaktierstellen der Adern des mehradrigen Kabels eine sich von der genannten Außenseite der Platine nach proximal wegerstreckende starre Anordnung bilden.

Insbesondere bei einem Einsatz dieses Bildaufnehmermoduls in einem Endoskop, das einen flexiblen Schaft aufweist, führt dies zu einer ungünstigen Verlängerung des starren distalen Schnabelteils des flexiblen Endoskops. Das starre Schnabelteil des flexiblen Endoskops muss bei Verwendung des bekannten Bildaufnehmermoduls nachteiligerweise länger ausgeführt werden.

Außerdem besteht bei diesem bekannten Bildaufnehmermodul der Nachteil, dass die elektronischen oder elektrischen Bauelemente in der Nut nicht in einer Ebene, sonders aus Platzgründen übereinander angeordnet sind. Die Folge ist ein hoher Zeit- und Kostenaufwand bei der Verdrahtung der dreidimensional angeordneten Bauteile und folglich bei der Fertigung des gesamten Bildaufnehmermoduls.

In DE-A-199 24 189 (und in der dazu inhaltsgleichen US 2002/0080233 A1) ist ein Bildaufnehmermodul beschrieben, dessen Platine aus einer entlang flexibler Verbindungsabschnitte faltbaren einteiligen Platte gebildet ist, die sich zu einem quaderförmigen, im Querschnitt im Wesentlichen U-förmigen Körper falten lässt. Der Platinenkörper weist im gefalteten Zustand zwei sich im Wesentlichen quer zum Bildsensor erstreckende und voneinander beabstandete Abschnitte und einen dritten Abschnitt auf, der im Wesentlichen parallel zum Bildsensor verläuft, wobei der Bildsensor an dem Ende des ersten und zweiten Abschnitts des Platinenkörpers angebracht ist, das von dem dritten Abschnitt beabstandet ist.

Ein aus US 5,754,313 bekanntes Bildaufnehmermodul weist zwei separate Platinen auf, wobei die beiden Platinen elektronische Miniaturbauteile aufnehmen und zur Kontaktierung des wegführenden Kabels bzw. Kabelsystems dienen. Die beiden Platinen verlaufen parallel zueinander und etwa rechtwinklig zur Fläche des Bildsensors. Der Zwischenraum zwischen den beiden einzelnen Platinen ist mit einer aushärtenden isolierenden Füllmasse ausgefüllt, und das mehradrige Kabel ist auf den Außenseiten der beiden Platinen kontaktiert. Sowohl bei dem aus DE 199 24 189 C2 als auch dem aus US 5,754,313 bekannten Bildaufnehmermodul erstrecken sich die Platinen senkrecht zum Bildsensor, so dass die Platinen dieser bekannten Bildaufnehmermodule axial sehr lang bauen, was, wie oben erwähnt, jedoch unerwünscht ist.

Das Gleiche gilt für ein aus US 5,857,963 bekanntes Bildaufnehmermodul, dessen Platinen bzw. Platinenteile sich senkrecht zum Bildsensor erstrecken. Ein weiteres damit vergleichbares Bildaufnehmermodul ist aus US 5,220,198 bekannt, bei dem die Platine flexibel ist und sich senkrecht zum Bildsensor erstreckt.

US 6,142,930 offenbart ein Bildaufnehmermodul mit einer flexiblen Platine, wobei die Adern des mehradrigen Kabels auf der dem Bildsensor abgewandten Außen- bzw. Unterseite der Platine kontaktiert sind, wodurch die oben erwähnten Nachteile einer Verlängerung des starren Teils des Bildaufnehmermoduls bestehen.

Ein weiterer Nachteil aller zuvor genannten Bildaufnehmermodule besteht in der schwierigen Assemblierung, d.h. der Kontaktierung und Integration elektronischer Bauelemente mit, bzw. an oder in der Platine.

Der Erfindung liegt die Aufgabe zugrunde, ein Bildaufnehmermodul der eingangs genannten Art dahingehend weiterzubilden, dass bei gleichzeitig kompakter Bauweise des Bildaufnehmermoduls mit starrer Platine, die sich parallel zum Bildsensor erstreckt, die Kontaktierung des mehradrigen Kabels nicht zu einer starren Verlängerung des Bildaufnehmermoduls in Längsrichtung führt.

Erfindungsgemäß wird diese Aufgabe hinsichtlich des eingangs genannten Bildaufnehmermoduls dadurch gelöst, dass sich die Kontaktierstellen der Adern auf der dem Bildsensor zugewandten Seite der Platine befinden, und die Adern mit ihren Ummantelungen von der dem Bildsensor abgewandten Seite zur dem Bildsensor zugewandten Seite durch die Platine durchgeführt sind.

Das erfindungsgemäße Bildaufnehmermodul weist somit wie das eingangs genannte bekannte Bildaufnehmermodul eine starre Platine auf, die parallel zum Bildsensor angeordnet ist, wodurch im Unterschied zu den weiteren im Stand der Technik bekannten Bildaufnehmermodulen die Baulänge in Längsrichtung klein bleibt. Im Unterschied zu dem eingangs genannten bekannten Bildaufnehmermodul wird jedoch das mehradrige Kabel nicht an der dem Bildsensor abgewandten Seite bzw. Unterseite der Platine kontaktiert, sondern die Kontaktierstellen befinden sich näher zum Bildsensor als die dem Bildsensor abgewandte Seite bzw. Unterseite der Platine. Hierdurch wird erreicht, dass auf Höhe der dem Bildsensor abgewandten Seite der Platine die einzelnen Adern des mehradrigen Kabels ihre Flexibilität vollständig entfalten können, wodurch die Anordnung aus Platine und Bildsensor an der dem Bildsensor abgewandten Seite der Platine relativ zu dem mehradrigen Kabel flexibel beweglich ist. Bei Koaxialkabeln als mehradrige Kabel sind es die Innenleiter, die an den vorstehend genannten Kontaktierstellen an der Platine kontaktiert sind, während die Außenleiter bzw. die Schirme an anderen Stellen kontaktiert sein können. Bei einem Einsatz des erfindungsgemäßen Bildaufnehmermoduls in einem flexiblen Endoskop kann das distale Schnabelteil daher kurz gebaut werden, wobei in die Länge des Schnabelteils nicht mehr die starre Kontaktierung des mehradrigen Kabels mit der Platine einbezogen werden muss.

Erfindungsgemäß befinden sich die Kontaktierstellen der Adern auf der dem Bildsensor zugewandten Seite der Platine, und die Adern sind von der dem Bildsensor abgewandten Seite zur dem Bildsensor zugewandten Seite durch die Platine durchgeführt.

Durch diese Ausgestaltung befinden sich die Kontaktierstellen der Adern des mehradrigen Kabels näher zum Bildsensor als die dem Bildsensor abgewandten Seite bzw. Unterseite der Platine, wodurch die oben beschriebenen Vorteile erreicht werden. Der weitere Vorteil dieser Maßnahme besteht darin, dass die Querabmessung der Platine durch die Adern nicht vergrößert wird, ohne dass auf der Außenseite Vertiefungen für die Aufnahme der Adern vorgesehen werden müssen.

Bei dem aus US 5,754,313 bekannten Bildaufnehmermodul ist das mehradrige Kabel zwar in Längsrichtung gesehen auf etwa halber Höhe an der Platine kontaktiert, jedoch ist diese Platine im Ausgangszustand flexibel, und wird erst durch das Ausfüllen mit einer aushärtbaren Füllmasse starr. Das Kontaktieren des mehradrigen Kabels an flexiblen Platinen oder Leiterplatten ist jedoch herstellungstechnisch aufwendig, und die Platine dieses bekannten Bildaufnehmermoduls baut, wie bereits erwähnt, in Längsrichtung sehr groß.

In einer bevorzugten Ausgestaltung ist die Platine einteilig ausgebildet.

Der Vorteil dieser Maßnahme liegt in einer einfachen Herstellbarkeit der Platine und einer geringen Anzahl an Bauteilen des Bildaufnehmermoduls.

Die Platine kann auch als Multilayerplatine ausgebildet sein, d.h. die Platine kann aus mehreren Lagen oder Schichten aufgebaut sein, die jeweils für sich Leiterbahnen aufweisen, wobei diese Leiterbahnen von Lage zu Lage oder Schicht zu Schicht vorzugsweise miteinander verbunden sind.

Als Alternative zu einer einteiligen oder einstückigen Ausgestaltung der Platine kann diese auch als Anordnung aus parallel zueinander und parallel zum Bildsensor verlaufenden starren Einzelplatinen mehrteilig ausgebildet sein.

Der Vorteil einer mehrteiligen Platine, die aus Einzelplatinen aufgebaut ist, besteht gegenüber der einteiligen bzw. einstückigen U-förmigen Platine des eingangs genannten bekannten Bildaufnehmermoduls darin, dass jede Einzelplatine zur Aufnahme und Kontaktierung von elektronischen Bauelementen dienen kann. Ausgehend von einer quaderförmigen Platine wird diese mit anderen Worten in vorzugsweise zwei, aber ggf. auch mehr Einzelplatinen aufgespalten, die eine Stapelanordnung in Längsrichtung des Bildaufnehmermoduls bilden. Bei dem bekannten Bildaufnehmermodul ist vorgesehen, elektronische Bauelemente in der U-förmigen Nut übereinander zu stapeln, was jedoch herstellungstechnisch hinsichtlich der Kontaktierung dieser Bauelemente schwierig zu realisieren ist, weil manche Kontaktierungen durch frei im Raum verlaufende Leiterdrähte realisiert werden müssen, was nur sehr schwierig und auch nur von Hand durchführbar ist. Demgegenüber kann bei der vorliegenden Ausgestaltung jede Einzelplatine beispielsweise ein elektronisches Bauelement aufnehmen, das dann unmittelbar auf der Einzelplatine kontaktiert werden kann, ohne dass eine "dreidimensionale" Kontaktierung erforderlich ist.

Dabei ist es weiterhin bevorzugt, wenn die Einzelplatinen über die Kontaktfinger des Bildsensors oder einzelne Leiter oder Leiterbahnen miteinander verbunden sind.

Ebenso ist es in diesem Zusammenhang bevorzugt, wenn die Einzelplatinen Vertiefungen in Form von Nuten und/oder Sacklochbohrungen zur Aufnahme von elektronischen Bauelementen aufweisen.

Hierbei ist von Vorteil, dass die elektronischen Bauelemente in den Vertiefungen platzsparend aufgenommen werden können, so dass die Einzelplatinen dann ohne oder mit nur geringem Abstand in Längsrichtung aufeinander gestapelt werden können.

In einer weiteren bevorzugten Ausgestaltung befinden sich die Kontaktierstellen der Adern auf einer dem Bildsensor zugewandten Seite der vom Bildsensor aus gesehen letzten Einzelplatine.

In dieser Ausgestaltung dient die letzte Einzelplatine daher vorrangig als Kabelterminal, während die eine oder mehreren weiteren Einzelplatinen dann zur Kontaktierung elektronischer Bauelemente dienen. Diese Verteilung der Funktionen der Kabelassemblierung und der Bauelementassemblierung auf einzelne Einzelplatinen verringert den Assemblierungsaufwand und macht die Assemblierung insbesondere einem automatisierten Prozess zugänglich. Nach Assemblierung aller Einzelplatinen müssen diese nur noch zusammengefügt und mit dem Bildsensor kontaktiert werden.

Wie bereits oben im Zusammenhang mit einer einstückigen oder einteiligen Platine beschrieben, ist es ebenso bevorzugt, wenn im Fall des Aufbaus der Platine aus mehreren Einzelplatinen zumindest eine der Einzelplatinen als Multilayerplatine ausgebildet ist.

Auch hier besteht der Vorteil in einer höheren Integrationsdichte und dadurch möglichen besonders kompakten Bauweise der Gesamtplatine.

In einer weiteren bevorzugten Ausgestaltung weist die Platine von der dem Bildsensor abgewandten Seite zu dem Bildsensor zugewandten Seite Durchkontaktierungen auf.

Diese Ausgestaltung ist insbesondere von Vorteil, wenn die Platine als Multilayerplatine ausgebildet ist, da über die Durchkontaktierungen dann die einzelnen Platinenlagen elektrisch miteinander kontaktiert werden können.

Im Fall des Aufbaus der Platine aus mehreren Einzelplatinen können diese vorzugsweise über die Kontaktfinger des Bildsensors miteinander verbunden werden, die vorzugsweise auf die dem Bildsensor abgewandte Seite der letzten Einzelplatine greifen, wodurch die Einzelplatinen über die Kontaktfinger auch mechanisch zusammengehalten werden.

Im Rahmen einer weiteren Ausgestaltung der zuvor genannten Maßnahme ist die Platine vorzugsweise von dem Bildsensor beabstandet.

Durch die Beabstandung des Bildsensors von der Platine steht ausreichend Raum für die Kontaktierstellen der Adern an der dem Bildsensor zugewandten Seite bzw. Oberseite der Platine zur Verfügung. Insbesondere können die Adern mit ihren Abschirmungen durch die Platine durchgeführt sein, wobei die freigelegten Drahtenden der Adern zur Kontaktierung mit der Oberseite der Platine U-förmig zur Platinenoberseite hin umgebogen sind.

In einer weiteren bevorzugten Ausgestaltung ist in dem Raum zwischen dem Bildsensor und der Platine zumindest ein elektronisches Bauelement angeordnet.

Hierbei ist von Vorteil, dass die Platine selbst nicht mit Vertiefungen wie oben beschrieben versehen werden muss, wodurch ein Arbeitsschritt eingespart wird, nämlich der Arbeitsschritt des Einfräsens von Vertiefungen in die Platine.

Dabei ist es weiterhin bevorzugt, wenn der Raum zwischen der Platine und dem Bildsensor mit einem aushärtbaren elektrisch isolierenden Füllmaterial ausgegossen ist.

Hierbei ist von Vorteil, dass das Füllmaterial einen Schutz für die elektronischen Bauelemente zwischen dem Bildsensor und der Platine bietet.

Im Rahmen der zuvor genannten Ausgestaltungen ist es weiterhin bevorzugt, wenn die Kontaktfinger des Bildsensors an zumindest einer Längsseite der Platine kontaktiert sind, an der vertieft angeordnete längliche Kontakte vorhanden sind.

Auch hier besteht wieder der Vorteil darin, dass die Kontaktfinger aufgrund der Vertiefungen ohne Erhöhung der Querschnittsabmessungen des Bildaufnehmermoduls mit der Platine kontaktiert werden können, wobei die länglichen Kontakte auch dazu dienen können, Leiterbahnen auf der dem Bildsensor zugewandten Seite der Platine als auch auf der dem Bildsensor abgewandten Seite der Platine miteinander elektrisch leitend zu verbinden, wodurch die dreidimensionale Struktur der Platine vorteilhaft ausgenutzt werden kann.

Gemäß einem weiteren Aspekt weist die Platine eine starre Basisplatine auf, die zur Kontaktierung des mehradrigen Kabels dient, und dass mit der Basisplatine zumindest ein erstes weiteres Platinenteil flexibel verbunden ist, das sich zumindest teilweise etwa senkrecht zur Basisplatine erstreckt.

Bei dieser Ausgestaltung dient die Basisplatine vorrangig der Kontaktierung des mehradrigen Kabels, und zwar auf der dem Bildsensor zugewandten Seite der Basisplatine, wie bereits oben beschrieben, um keine starre Verlängerung des Bildaufnehmermoduls zu erhalten. Die Basisplatine kann aufgrund des zumindest einen weiteren Platinenteils in Längsrichtung gerade so dick ausgeführt werden, dass sie starr ist, um die Kontaktierung des mehradrigen Kabels herstellungstechnisch einfach durchführen zu können. Das zumindest eine weitere Platinenteil, das sich zumindest teilweise etwas senkrecht zur Basisplatine erstreckt, kann dann, wie in einer weiteren bevorzugten Ausgestaltung vorgesehen ist, zur Befestigung und Kontaktierung zumindest eines elektronischen Bauelements dienen.

Durch die flexible Anbindung des zumindest einen weiteren Platinenteils an der Basisplatine kann die Anordnung aus Basisplatine und dem zumindest einen weiteren Platinenteil vor der Kontaktierung der elektrischen Bauelemente flach ausgebreitet werden, wodurch die Anbringung und Kontaktierung der elektronischen Bauelemente insbesondere automatisiert erfolgen kann.

In einer weiteren bevorzugten Ausgestaltung weist das zumindest eine erste weitere Platinenteil zumindest einen ersten Abschnitt auf, der sich etwa senkrecht von der Basisplatine weg zum Bildsensor hin erstreckt, und zumindest einen zweiten Abschnitt, der sich an den ersten Abschnitt anschließt und etwa parallel zur Basisplatine erstreckt.

Hierbei ist von Vorteil, dass der erste Abschnitt und der zweite Abschnitt elektronische Bauelemente tragen und einhäusen können, insbesondere, wenn noch ein dritter Abschnitt vorgesehen ist, der parallel zum ersten Abschnitt verläuft, um bei gleichzeitig kompakter Bauweise den Raum zwischen der Basisplatine und dem weiteren Platinenteil optimal zu nutzen.

In einer weiteren bevorzugten Ausgestaltung dieses Aspekts ist die Basisplatine mit zumindest zwei weiteren zweiten und dritten Platinenteilen flexibel verbunden, die von zwei gegenüberliegenden Längsseiten der Basisplatine senkrecht zu dieser wegführen und das mehradrige Kabel einfassen.

Hierbei ist von Vorteil, dass das mehradrige Kabel durch die zumindest zwei weiteren zweiten und dritten Platinenteile geschützt eingefasst ist, wobei durch die flexible Verbindung der zwei weiteren Platinenteile mit der Basisplatine keine Beeinträchtigung der Flexibilität des Bildaufnehmermoduls ab der Unterseite der Basisplatine einhergeht. Die zwei weiteren zweiten und dritten Platinenteile können weitere Leiterbahnen aufweisen, und können insbesondere auch zur außenseitigen Kontaktierung der Kontaktfinger des Bildsensors dienen.

In einer weiteren bevorzugten Ausgestaltung sind die Basisplatine und das zumindest eine erste und gegebenenfalls zumindest eine zweite und dritte weitere Platinenteil mit der Basisplatine aus einem Platinenrohling gefertigt, wobei die Basisplatine gegenüber der oder den weiteren Platinenteilen eine größere Materialstärke aufweist.

Hierbei ist von Vorteil, dass die Gesamtplatine aus Basisplatine und den weiteren Platinenteilen zur Kontaktierung der elektronischen Bauelemente zunächst flach ausgebreitet werden kann, was die Kontaktierung der Bauelemente insbesondere einem automatisierten Prozess der Beschickung und/oder Kontaktierung zugänglich macht, während zum Schluss nur noch der Bildsensor als Letztes auf die entsprechend in Form gefaltete Platine aufgebracht werden muss. Im Unterschied zu den im Stand der Technik bekannten faltbaren Platinen ist die Basisplatine, die als Kabelterminal dient, jedoch starr, was die Kontaktierung des mehradrigen Kabels wesentlich vereinfacht. Die größere Materialstärke der Basisplatine gegenüber den weiteren Platinenteilen kann beispielsweise dadurch hergestellt werden, dass mit Ausnahme der Basisplatine von den Platinenteilen das Trägermaterial des Platinenrohlings entfernt wird, oder umgekehrt an die Basisplatine Trägermaterial angefügt wird.

Weitere Vorteile und Merkmale ergeben sich aus der nachfolgenden Beschreibung der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig 1a)+b): ein Beispiel eines Bildaufnehmermoduls, wobei das Bildaufnehmermodul in Fig. 1a) in perspektivischer Seitenansicht im zu- sammengesetzten Zustand und in Fig. 1b) in auseinandergezogener An- ordnung der einzelnen Komponenten des Bildaufnehmermoduls gezeigt ist;
- Fig. 2a)+b): ein weiteres Beispiel eines Bildaufnehmermoduls, wobei das Bildaufnehmermodul in Fig. 2a) in perspektivischer Seitenansicht im zu- sammengesetzten Zustand und in Fig. 2b) in auseinandergezogener An- ordnung der einzelnen Komponenten des Bildaufnehmermoduls gezeigt ist;
- Fig. 3a)+b): ein weiteres Beispiel eines Bildaufnehmermoduls, wobei das Bildaufnehmermodul in Fig. 3a) in perspektivischer Seitenansicht in zu- sammengesetztem Zustand und in Fig. 3b) in auseinandergezogener An- ordnung der einzelnen Komponenten des Bildaufnehmermoduls gezeigt ist;
- Fig. 4a)+b): ein weiteres Beispiel eines Bildaufnehmermoduls, wobei das Bildaufnehmermodul in Fig. 4a) in perspektivischer Seitenansicht in zu- sammengesetztem Zustand und in Fig. 4b) in auseinandergezogener An- ordnung der einzelnen Komponenten des Bildaufnehmermoduls gezeigt ist;
- Fig. 5a)+ b): ein weiteres Beispiel eines Bildaufnehmermoduls, wobei das Bildaufnehmermodul in Fig. 5a) in perspektivischer Seitenansicht in zu- sammengesetztem Zustand und in Fig. 5b) in auseinandergezogener An- ordnung der einzelnen Komponenten des Bildaufnehmermoduls gezeigt ist;
- Fig. 6a)+b): zwei verschiedene perspektivische Ansichten einer Platine, die in einem Bildaufnehmermodul verwendet werden kann;
- Fig. 7a)-c): ein erstes Ausführungsbeispiel eines Bildaufnehmermoduls, wobei Fig. 7a) eine perspektivische Ansicht, Fig. 7b) eine Ansicht auf die Unterseite und Fig. 7c) eine Seitenansicht des Bildaufnehmermoduls zeigt;
- Fig. 8a)-c): ein weiteres Ausführungsbeispiel eines Bildaufnehmermoduls, wobei Fig. 8a) eine perspektivische Ansicht, Fig. 8b) eine Ansicht auf die Unterseite des Bildaufnehmermoduls und Fig. 8c) eine Seitenansicht des Bildauf- nehmermoduls zeigt;
- Fig. 9a)+b): ein weiteres Ausführungsbeispiel eines Bildaufnehmermoduls, wobei Fig. 9a) eine perspektivische Ansicht und Fig. 9b) eine Seitenansicht zeigt; und
- Fig. 10a)+b): ein weiteres Ausführungsbeispiel eines Bildaufnehmermoduls, wobei Fig. 10a) eine perspektivische Ansicht und Fig. 10b) eine Seitenansicht zeigt.

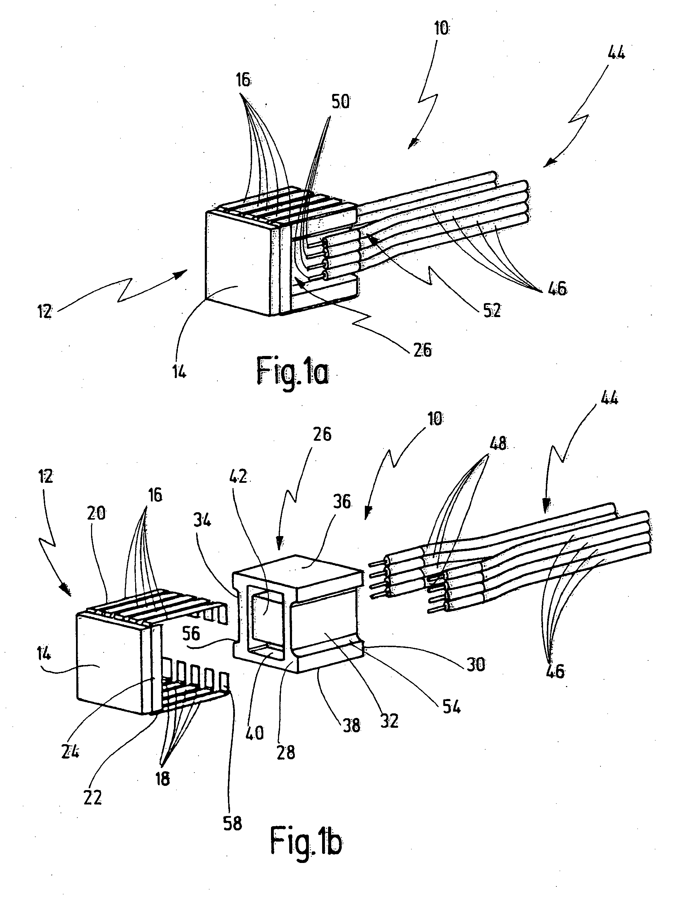

In Fig. 1 bis 5 sowie 7 bis 10 sind verschiedene Beispiele und Ausführungsbeispiele von Bildaufnehmermodulen dargestellt, die nachfolgend im Einzelnen beschrieben werden.

Alle gezeigten Bildaufnehmermodule eignen sich insbesondere für einen Einbau in ein Endoskop, insbesondere in ein flexibles Endoskop, wobei die Bildaufnehmermodule zum Einbau in die distale Spitze des Schafts eines solchen Endoskops, das auch als Schnabelteil bezeichnet wird, aufgrund ihrer axial kurz bauenden Länge geeignet sind.

Die gezeigten Bildaufnehmermodule sind opto-elektronische Bauteile in miniaturisierter Form. Es versteht sich, dass die Darstellungen in Fig. 1 bis 10 stark vergrößert sind. Beispielhafte Querschnittsabmessungen und Längen liegen im Bereich von 1 bis 4 Millimetern.

In Fig. 1a) und b) ist ein mit dem allgemeinen Bezugszeichen 10 versehenes Bildaufnehmermodul gemäß einem ersten Beispiel gezeigt.

Das Bildaufnehmermodul 10 weist als erste Hauptkomponente einen elektronischen Bildsensor 12 auf. Der Bildsensor 12 weist eine lichteintrittsseitige Außenseite 14 auf, durch die Licht in den Bildsensor 12 eintritt. Der lichteintrittsseitigen Außenseite 14 ist im Gebrauch des Bildaufnehmermoduls 10, beispielsweise in einem Endoskop in dessen distaler Spitze eingebautem Zustand, eine Abbildungsoptik vorgeschaltet, um ein zu beobachtendes Objekt auf dem Bildsensor 12 abzubilden.

Der Bildsensor 12 ist in CCD- oder CMOS-Technologie in TAB-Konfiguration ausgebildet.

Der Bildsensor 12 weist eine Mehrzahl von Kontaktfingern 16 und 18 auf. In dem gezeigten Ausführungsbeispiel weist der Bildsensor 12 insgesamt 10 Kontaktfinger 16, 18 auf. Die Kontaktfinger 16 und 18 sind in zwei Reihen an gegenüberliegenden Seiten 20 und 22 eines Basiskörpers 24 des Bildsensors 12 angeordnet. Die Kontaktfinger 16 bilden dabei eine erste Reihe von Kontaktfingern und die Kontaktfinger 18 eine zweite Reihe von Kontaktfingern.

Eine weitere Hauptkomponente des Bildaufnehmermoduls 10 ist eine Platine 26, mit der die Kontaktfinger 16, 18 im zusammengesetzten Zustand des Bildaufnehmermoduls 10 gemäß Fig. 1a) elektrisch kontaktiert sind.

Die Platine 26 ist in dem gezeigten Ausführungsbeispiel einstückig und einteilig ausgebildet. Des weiteren ist die Platine 26 starr. Die Platine 26 weist eine dem Bildsensor 12 zugewandte Seite 28 und eine vom Bildsensor 12 abgewandte Seite 30 auf. Die Platine 26 ist insgesamt im Allgemeinen quaderförmig ausgebildet. Die Platine 26 weist weiterhin vier geschlossene Längsseiten 32, 34, die einander gegenüberliegen, sowie 36 und 38 auf, die ebenfalls einander gegenüberliegen. Die dem Bildsensor 12 zugewandte Seite 28 ist parallel zum Bildsensor 12 ausgerichtet, ebenso wie die dem Bildsensor 12 abgewandte Seite 30. Die Seite 30 wird in der vorliegenden Beschreibung auch als Unterseite der Platine 26 bezeichnet.

Die Platine 26 ist insgesamt parallel zum Bildsensor 12 ausgerichtet, wobei sich die Seiten 32 bis 38 in Richtung senkrecht zur Außenseite 14 des Bildsensors 12 erstrecken, die die Längsrichtung des Bildaufnehmermoduls 10 definiert.

Die Platine 26 kann aus einem Vollmaterial hergestellt werden. Auf der dem Bildsensor 12 zugewandten Seite 28 weist die Platine 26 zwischen den vier Längsseiten 32 bis 38 eine Ausfräsung in Form einer Sacklochbohrung 40 auf. In der Sacklochbohrung 40 ist ein elektronisches Bauelement 42 angeordnet, wobei in der Sacklochbohrung 40 auch mehrere solcher elektronischer Bauelemente angeordnet sein können. Wie in Fig. 1b) dargestellt ist, ist das Bauelement 42 in der Sacklochbohrung 40 vollständig aufgenommen, d.h. steht nicht über die Seite 28 der Platine 26 vor, wodurch im zusammengesetzten Zustand des Bildaufnehmermoduls 10 in Fig. 1a) die Seite 28 unmittelbar an den Basiskörper 24 angesetzt werden kann.

Das oder die elektronischen Bauelemente 42 sind Bestandteil einer Steuerelektronik für den Bildsensor 12.

Die Platine 26 weist nicht dargestellte Kontaktierungen zur Kontaktierung des oder der elektronischen Bauelemente 42 auf.

Die Platine 26 dient des Weiteren zur Kontaktierung eines mehradrigen Kabels 44, das in dem gezeigten Ausführungsbeispiel insgesamt acht Adern 46, 48 aufweist.

Die Adern 46, 48 des Kabels 44 sind mit der Platine 26 an Kontaktierungsstellen 50, wie für die Adern 46 gezeigt ist, kontaktiert, die näher zum Bildsensor 12 liegen als die dem Bildsensor 12 abgewandte Seite bzw. Unterseite 30 der Platine 26.

Die Kontaktierungsstellen 50 befinden sich bei diesem Ausführungsbeispiel an den Längsseiten 32 und 34 der Platine 26. Aufgrund der näher zum Bildsensor 12 verlagerten Kontaktierung der Adern 46, 48 werden auf der Unterseite 30 der Platine 26 starre Kontaktierungsstellen vermieden, bzw. mit anderen Worten ist der Übergang der Adern 46, 48 an einer Stelle 52 gemäß Fig. 1a) zur Platine 26, also unmittelbar an der Unterseite 30 der Platine 26, flexibel, was insbesondere für den Einbau des Bildaufnehmermoduls 10 in die distale Spitze eines flexiblen Endoskops vorteilhaft ist, weil die distale Spitze bzw. das Schnabelteil des flexiblen Endoskops axial sehr kurz ausgeführt werden kann.

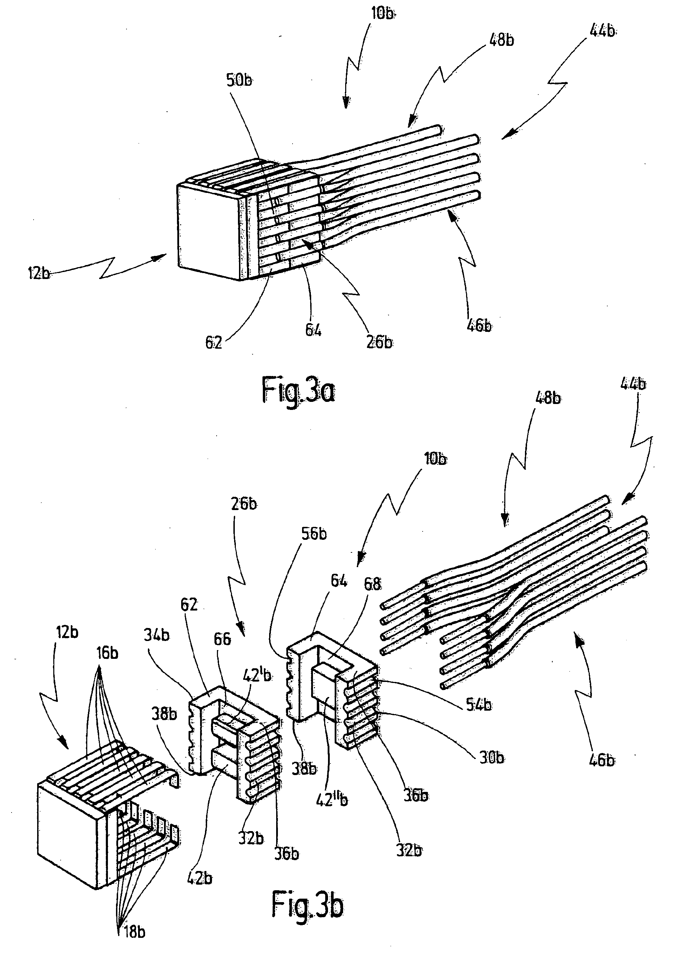

Damit die Adern 46 bzw. 48 auf den Längsseiten 32 und 34 nicht auftragen bzw. die Querabmessung des Bildsensors 12 in diesen Richtungen übersteigen, sind in den Längsseiten 32 und 34 entsprechende Vertiefungen 54 und 56 ausgespart, in denen die Adern 46, 48 aufgenommen sind. Anstelle einer einzigen Vertiefung 54 für die vier Adern 46 bzw. einer einzigen Vertiefung 56 für die vier Adern 48 können auch einzelne Vertiefungen für die einzelnen Adern 46, 48 vorgesehen werden, wie beispielsweise bei dem Ausführungsbeispiel in Fig. 3 gezeigt ist.

Wie aus Fig. 1 hervorgeht, werden die Adern 46, 48 an den Längsseiten 32 und 34 der Platine 26 kontaktiert, die frei von den Kontaktfingern 16, 18 des Bildsensors 12 sind. Die Kontaktfinger 16, 18 erstrecken sich entlang der Längsseiten 36, 38 der Platine 26 und greifen auf die Unterseite 30 der Platine 26, wo sie vorzugsweise kontaktiert sind. Enden 58 der Kontaktfinger 26, 18 sind entsprechend auf die Unterseite 30 der Platine 26 umgebogen.

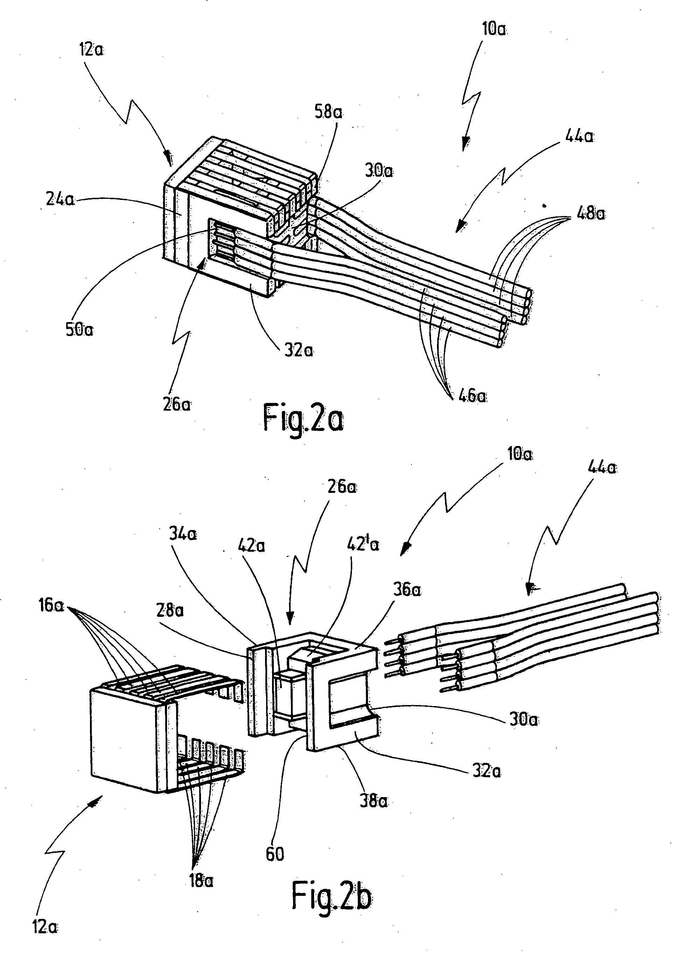

In Fig. 2a) und b) ist ein weiteres Beispiel eines Bildaufnehmermoduls 10a dargestellt, wobei einzelne Teile des Bildaufnehmermoduls 10a, die gleich oder vergleichbar mit den entsprechenden Teilen des Bildaufnehmermoduls 10 sind, mit den gleichen Bezugszeichen, ergänzt durch den Buchstaben a, versehen sind.

Nachfolgend werden vorrangig die Unterschiede des Bildaufnehmermoduls 10a zu dem Bildaufnehmermodul 10 beschrieben. Sofern nichts Gegenteiliges ausgeführt wird, gilt für das Bildaufnehmermodul 10a die Beschreibung des Bildaufnehmermoduls 10.

Die Platine 26a des Bildaufnehmermoduls 10a ist zwar wiederum einstückig und einteilig wie die Platine 26 ausgebildet, weist jedoch im Unterschied zu der Platine 26 die Form eines U auf. Die Platine 26a weist entsprechend eine sich zwischen zwei Längsseiten 36a und 38a erstreckende Nut 60 auf, die an den Längsseiten 36a und 38a offen ist.

In der durch die Nut 60 gebildeten Vertiefung sind elektronische Bauelemente 42a und 42'a angeordnet, und zwar in dreidimensionaler Anordnung, d.h. die Bauelemente 42a und 42'a sind übereinander gestapelt in der Nut 60 angeordnet, wie dies in DE 10 2004 056 946 A1 beschrieben ist, wobei auf jenes Dokument bezüglich weiterer Einzelheiten verwiesen wird.

Die Adern 46a und 48a des flexiblen mehradrigen Kabels 44a sind an den geschlossenen Längsseiten 34a an Kontaktierungsstellen 50a kontaktiert, die näher zum Bildsensor 12a liegen als die Unterseite 30a der Platine 26a. Die Kontaktfinger 16a und 18a des Bildsensors 12a erstrecken sich entlang der offenen Längsseiten 36a und 38a der Platine 26a und sind mit ihren Enden 58a auf der Unterseite 30a der Platine 26a kontaktiert.

Fig. 3 zeigt ein weiteres Beispiel eines Bildaufnehmermoduls 10b, das gegenüber den Ausführungsbeispielen in Fig. 1 und 2 weiter abgewandelt ist. Teile des Bildaufnehmermoduls 10b, die mit den entsprechenden Teilen des Bildaufnehmermoduls 10 gleich oder vergleichbar sind, sind mit den gleichen Bezugszeichen, ergänzt durch den Buchstaben b, versehen.

Das Bildaufnehmermodul 10b weist eine Platine 26b auf, die im Unterschied zu den Platinen 26 und 26a nicht einstückig und einteilig ausgebildet ist, sondern mehrteilig, und zwar wird die Platine 26b durch eine Anordnung aus parallel zueinander verlaufenden starren Einzelplatinen 62 und 64 gebildet, die im zusammengesetzten Zustand gemäß Fig. 3a) vorzugweise einander berührend dicht zusammengefügt sind.

Die Einzelplatinen 62 und 64 sind beide vergleichbar zu der Platine 26a U-förmig ausgebildet, wobei jedoch auch eine Ausführung der Einzelplatinen 62 und 64 mit einer Sacklochbohrung wie bei der Platine 26 möglich ist.

In dem gezeigten Beispiel nehmen beide Einzelplatinen 62 und 64 jeweils ein oder mehrere elektronische Bauelemente 42b, 42'b (Einzelplatine 62) und 42"b (Einzelplatine 64) auf. Im Unterschied zu der Platine 26a, bei der die Bauelemente 42a, 42'a übereinandergestapelt in der Nut 60 der Platine 26a angeordnet sind, sind die Bauelemente 42b, 42'b und 42"b jeweils nur in "zweidimensionaler" Anordnung in den Einzelplatinen 62 und 64 aufgenommen. Die Bauelemente 42b, 42'b und 42"b können somit jeweils an einem Boden 66 bzw. 68 der Einzelplatinen 62, 64 montiert und kontaktiert werden, wodurch die Assemblierung der Einzelplatinen 62, 64 mit den Bauelementen 42b, 42'b, 42"b einfacher ist als die Assemblierung der Platine 26a mit den Bauelementen 42a, 42'a.

Die Kontaktfinger 16b und 18b des Bildsensors 12b sind auf einer dem Bildsensor 12b abgewandten Unterseite 30b der Platine 26b, genauer gesagt der Einzelplatine 64, kontaktiert. Die Kontaktfinger 16b, 18b übergreifen somit die Einzelplatinen 62, 64 an deren Längsseiten 36b bzw. 38b und können insbesondere dazu dienen, die Einzelplatinen 62 und 64 aneinander zusammenzuhalten.

Die Kontaktierungsstellen 50b der Adern 44b, 46b befinden sich von der Unterseite 30b der Platine 26b aus gesehen zum Bildsensor 12b hin versetzt an Längsseiten 32b bzw. 34b, die frei von den Kontaktfingern 16b, 18b sind, wobei die Adern 44b, 46b, wie in Fig. 3a) gezeigt ist, die Verbindungsstelle zwischen den Einzelplatinen 62, 64 überbrücken.

Die Einzelplatinen 62, 64 können des Weiteren auch über einzelne Leiter oder Leiterbahnen miteinander elektrisch verbunden sein, um die Integrationsdichte der Platine 26b auf möglichst geringem Raum zu erhöhen.

Die Platine 26b des Bildaufnehmermoduls 10b weist als weiteren Unterschied zu dem Bildaufnehmermodul 10 bzw. 10a für jede der Adern 44b, 46b separate Vertiefungen 54b bzw. 56b auf.

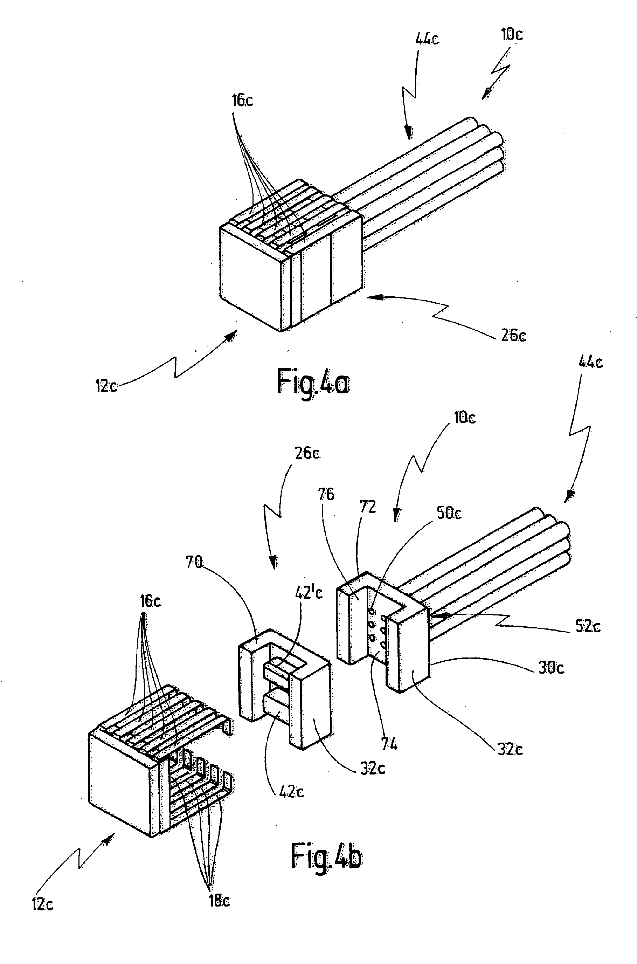

In Fig. 4 ist ein weiteres Beispiel eines Bildaufnehmermoduls 10c gezeigt, wobei Teile des Bildaufnehmermoduls 10c, die mit entsprechenden Teilen des Bildaufnehmermoduls 10 gleich oder vergleichbar sind, mit den gleichen Bezugszeichen, ergänzt durch den Buchstaben c, versehen sind.

Das Bildaufnehmermodul 10c weist eine Platine 26c auf, die wie bei dem Bildaufnehmermodul 10b aus zwei Einzelplatinen 70, 72 gebildet wird, wobei beide Einzelplatinen 70, 72 U-förmig ausgebildet sind. Auch hier kann anstelle einer U-förmigen Ausgestaltung der Einzelplatinen 70, 72 auch eine Ausgestaltung mit Sacklochbohrungen oder dergleichen in Betracht gezogen werden. Es versteht sich, dass die Nuten der Einzelplatinen anstatt parallel auch um 90° verdreht zueinander ausgerichtet sein können.

Im Unterschied zu der Platine 26b nimmt die Einzelplatine 72, d.h. die vom Bildsensor 12c aus gesehen letzte Einzelplatine die Funktion der Kontaktierung des mehradrigen Kabels 44c ein. Die Adern des mehradrigen Kabels 44c sind mit der Platine 26c, genauer gesagt mit der Einzelplatine 72 jedoch nicht an einer dem Bildsensor 12c abgewandten Unterseite 30c der Einzelplatine 72 kontaktiert, wie dies bei dem Bildaufnehmermodul gemäß DE 10 2004 056 946 A1 der Fall ist, sondern auf einer dem Bildsensor 12c zugewandten 74. Die Adern des Kabels 44c sind dabei von der Seite 30c zur Seite 74 durch die Einzelplatine 72 durchgeführt, so dass sich die Kontaktierstellen 50c näher am Bildsensor 12c befinden als die Unterseite 30c. Auch hierdurch wird wiederum erreicht, dass der unmittelbare Übergang vom Kabel 44c zur Unterseite 30c der Platine 26c an der Stelle 52c flexibel ist.

Während nur die Einzelplatine 70 gemäß Fig. 4b) mit elektronischen Bauelementen 42c, 42'c versehen ist, kann jedoch auch die Einzelplatine 72 zusätzlich derartige elektronische Bauelemente in ihrer nutartigen Vertiefung 76 aufnehmen.

Im Übrigen entspricht das Bildaufnehmermodul 10c dem Bildaufnehmermodul 10b.

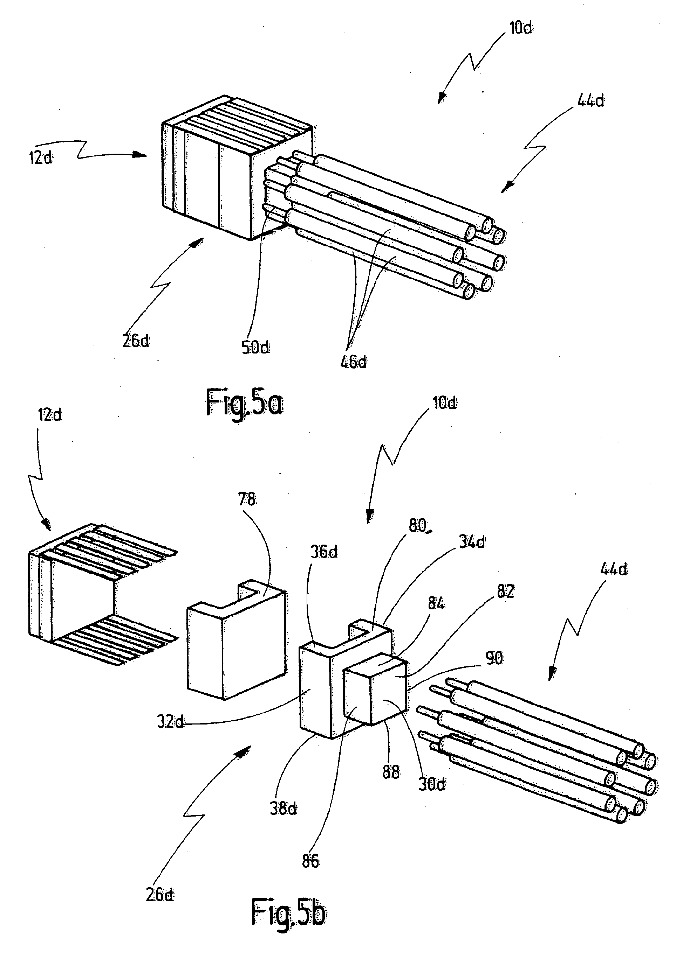

Eine weitere Abwandlung des Bildaufnehmermoduls 10b in Fig. 3 ist in Form eines Bildaufnehmermoduls 10d in Fig. 5 gezeigt. Teile des Bildaufnehmermoduls 10d, die mit entsprechenden Teilen des Bildaufnehmermoduls 10 gleich oder vergleichbar sind, sind mit den gleichen Bezugszeichen, ergänzt durch den Buchstaben d, versehen.

Das Bildaufnehmermodul 10d weist eine Platine 26d auf, die wie bei den beiden vorhergehenden Ausführungsbeispielen aus zwei Einzelplatinen 78 und 80 gebildet wird.

Die Einzelplatine 80, d.h. die vom Bildsensor 12d aus gesehen letzte Einzelplatine dient als Kabelterminal zum Kontaktieren der Adern des Kabels 44d. Im Unterschied zu dem vorhergehenden Ausführungsbeispiel weist die Einzelplatine 80 einen Abschnitt 82 auf, der Längsseiten 84, 86, 88, 90 aufweist, von denen zumindest eine, im gezeigten Beispiel alle gegenüber den Längsseiten 32d, 34d, 36d, 38d des übrigen Teils der Platine 26d zurückversetzt sind, so dass der Abschnitt 82 eine geringere Querschnittsabmessung aufweist als der übrige Teil der Platine 26d und als der Bildsensor 12d.

Zumindest eine, im gezeigten Beispiel alle Längsseiten 84, 86, 88 und 90 dienen zur Kontaktierung der Adern des Kabels 44d, wobei auch hierbei die Kontaktierstellen 50d von der vom Bildsensor 12d abgewandten Unterseite 30d in Richtung zum Bildsensor 12d hin beabstandet sind.

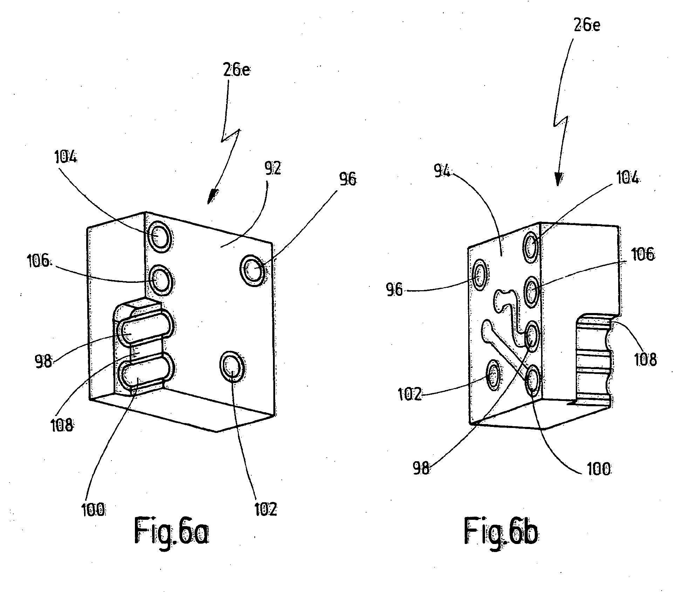

In Fig. 6 ist eine weitere Platine 26e dargestellt, die als Einzelplatine in den Beispielen gemäß Fig. 3 bis 5 anstelle einer der dort dargestellten Einzelplatinen verwendet werden kann.

Die Platine 26e weist von einer ersten Seite 92 zu einer zweiten Seite 94 reichende Durchkontaktierungen 94, 96, 98, 100, 102, 104, 106 auf, wobei die Platine im Bereich mancher Durchkontaktierungen, wie bei den Durchkontaktierungen 98 und 100, eine oder mehrere Ausnehmungen bzw. Abfräsungen 108 aufweisen kann.

Die Durchkontaktierungen 96 bis 106 sind mit leitfähigem Material ausgekleidet.

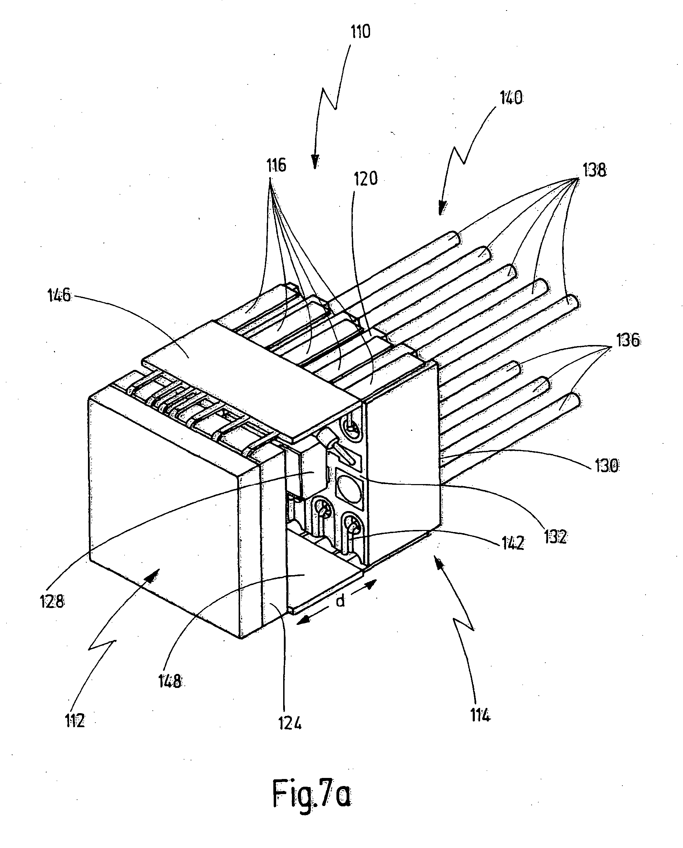

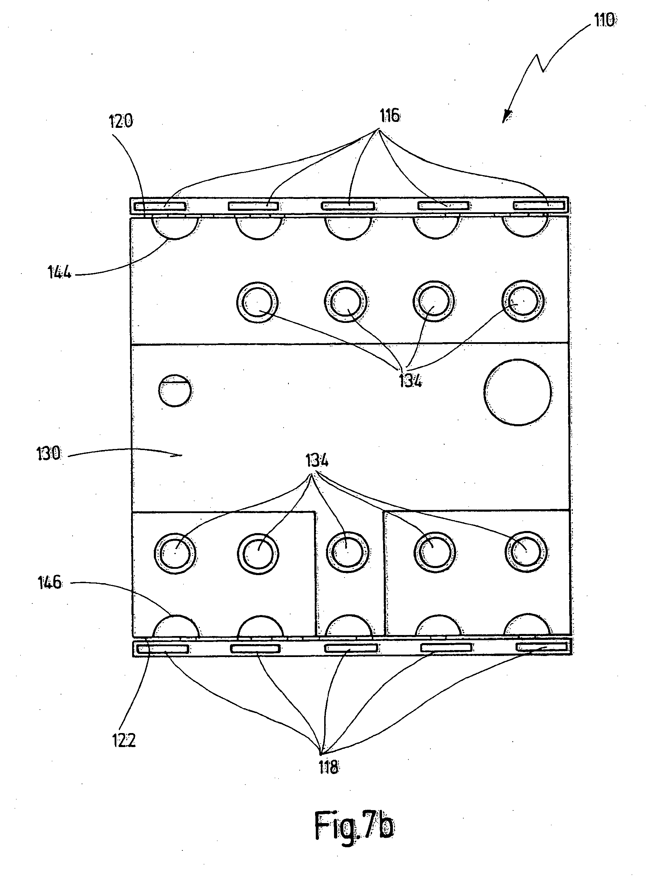

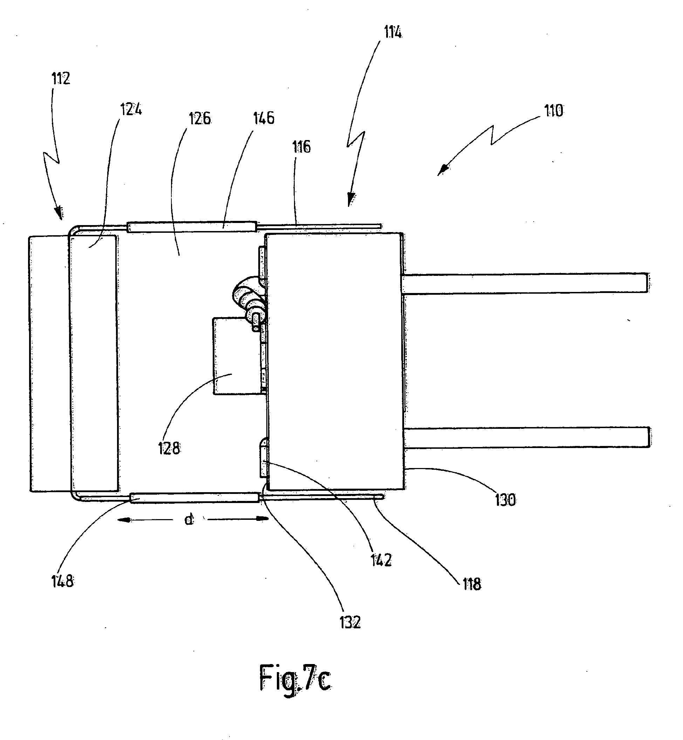

In Fig. 7 bis 10 sind weitere Bildaufnehmermodule dargestellt, anhand derer weitere Aspekte der Erfindung beschrieben werden.

In Fig. 7a) bis c) ist ein Bildaufnehmermodul 110 dargestellt, mit einem Bildsensor 112 und mit einer Platine 114, mit der eine erste Reihe von Kontaktfingern 116 und eine zweite Reihe von Kontaktfingern 118 (vergleiche Fig. 7b)) des Bildsensors 112 elektrisch kontaktiert sind, wobei sich die Kontaktfinger 116, 118 entlang von Längsseiten 120, 122 der Platine 114 erstrecken.

Bei dem gezeigten Ausführungsbeispiel des Bildaufnehmermoduls 110 ist die Platine 114 vergleichbar mit der Platine 26 oder 26a einstückig bzw. einteilig ausgebildet. Die Platine 114 ist parallel zum Bildsensor 112 angeordnet und weist in Längsrichtung gesehen eine Länge auf, die gegenüber der Länge der Platine 26 bzw. 26a etwa halbiert ist.

Im Unterschied zu der Platine 26 oder 26a ist die Platine 114 von dem Bildsensor 112, genauer gesagt von dessen Basiskörper 124 um einen Abstand d beabstandet, so dass zwischen dem Basiskörper 124 des Bildsensors 112 und der Platine 114 ein freier Raum 126 vorhanden ist, der gegebenenfalls mit einer aushärtbaren Füllmasse, die elektrisch isolierend ist, ausgegossen sein kann. In dem Raum zwischen dem Bildsensor 112 und der Platine 114 ist zumindest ein elektronisches Bauelement 128 auf der Platine 114 angeordnet und mit dieser elektrisch kontaktiert.

Vergleichbar zu der Platine 26e in Fig. 6 weist die Platine von ihrer dem Bildsensor 112 abgewandten Seite bzw. Unterseite 130 ausgehende und bis zu der dem Bildsensor 112 zugewandten Seite oder Oberseite 132 reichende Bohrungen 134 auf, durch die Adern 136, 138 eines mehradrigen flexiblen Kabels 140 durchgeführt sind, wobei die einzelnen Adern auf der dem Bildsensor 112 zugewandten Seite 132 der Platine 114 mit dieser kontaktiert sind. Die Kontaktierstellen, wie beispielsweise für eine Kontaktierstelle 142 einer der Adern 136 in Fig. 7a) gezeigt ist, befinden sich somit ebenfalls wiederum näher zum Bildsensor 112 als die dem Bildsensor 112 abgewandte Unterseite 130 der Platine 114.

Die Kontaktfinger 116, 118 des Bildsensors 112 sind, wie bereits erwähnt, entlang der Längsseiten 120, 122 der Platine 114 angeordnet und an diesen beiden Längsseiten 120, 122 mit der Platine 114 elektrisch kontaktiert. An den Längsseiten 120, 122 der Platine 114 sind eine Mehrzahl von vertieft angeordneten länglichen Kontakten 144 (im gezeigten Ausführungsbeispiel insgesamt zehn) angeordnet, die sich in Längsrichtung der Platine 114 erstrecken und von der Unterseite 130 bis zur Oberseite 132 reichen. Mit diesen länglichen Kontakten 144, 146 sind die Kontaktfinger 116 bzw. 118 elektrisch kontaktiert.

Die Kontaktfinger 116 bzw. 118 sind jeweils durch eine Platte 146 bzw. 148 stabilisiert, insbesondere in dem Bereich, in dem die Kontaktfinger 116, 118 den freien Raum 126 zwischen der Platine 114 und dem Bildsensor 112 überbrücken. Die Platten 146, 148 sind aus elektrisch isolierendem Material.

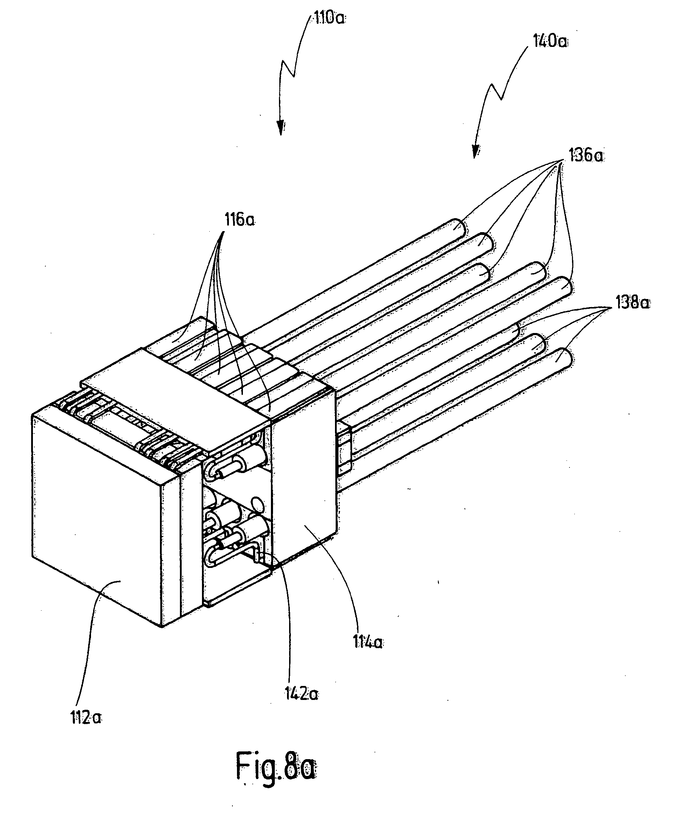

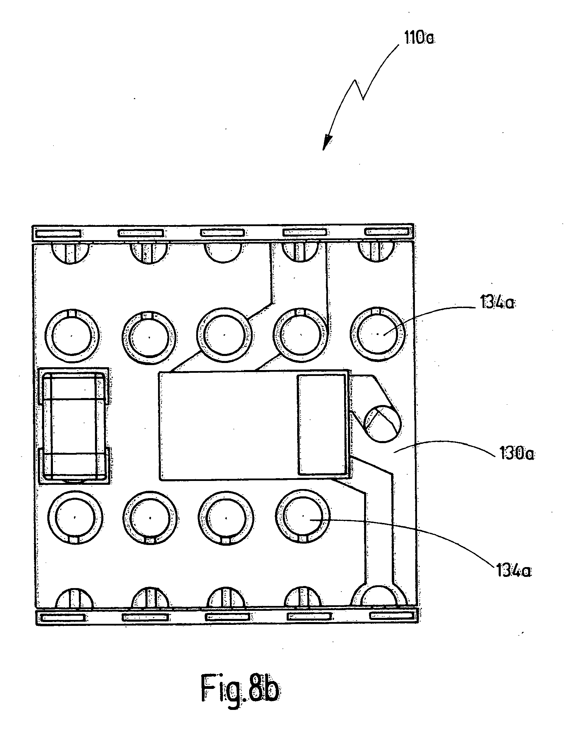

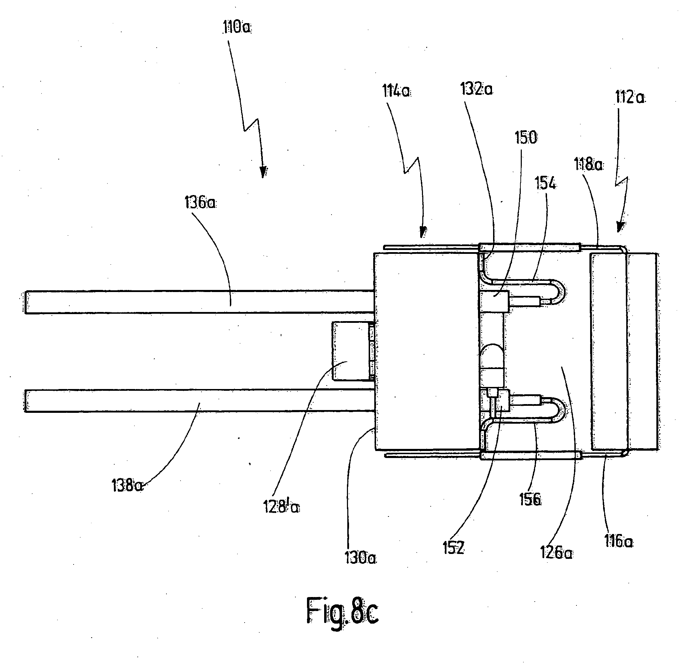

In Fig. 8a) bis c) ist eine Abwandlung des Bildaufnehmermoduls 110 in Form eines Bildaufnehmermoduls 110a dargestellt. Teile des Bildaufnehmermoduls 110a, die mit entsprechenden Teilen des Bildaufnehmermoduls 110 gleich oder vergleichbar sind, sind mit den gleichen Bezugszeichen, ergänzt durch den Buchstaben a, versehen.

Das Bildaufnehmermodul 110a weist eine Platine 114a auf, die von dem Bildsensor 112a wie bei dem vorherigen Ausführungsbeispiel beabstandet ist, so dass zwischen dem Bildsensor 112a und der Platine 114a ein freier Raum 126a vorhanden ist, der gegebenenfalls mit einer elektrisch isolierenden aushärtbaren Füllmasse ausgegossen ist.

Im Unterschied zu dem vorherigen Ausführungsbeispiel ist zumindest auch ein elektronisches Bauelement 128'a auf der dem Bildsensor 112a abgewandten Unterseite 130a der Platine 114a angeordnet und mit dieser elektrisch kontaktiert.

Die Adern 136a und 138a des mehradrigen Kabels 140a sind wiederum durch Bohrungen 134a durch die Platine 114a durchgeführt und auf einer Oberseite 132a mit der Platine 114a kontaktiert.

Die Adern 136a und 138a sind gegenüber dem vorherigen Ausführungsbeispiel so weit durch die Platine 114a durchgeführt, dass sie mit ihren Ummantelungen, insbesondere Abschirmungen 150 bzw. 152 aus der Oberseite 132a der Platine 114 vorstehen, wobei die freiliegenden Enden 154 bzw. 156 aus der Längsrichtung um 180° umgebogen und ihre äußersten Enden auf der Oberseite 132a mit der Platine 114a elektrisch kontaktiert sind.

Es versteht sich, dass auch auf der Oberseite 132a der Platine 114a elektrische Bauelemente angeordnet und mit der Platine 114a kontaktiert sein können.

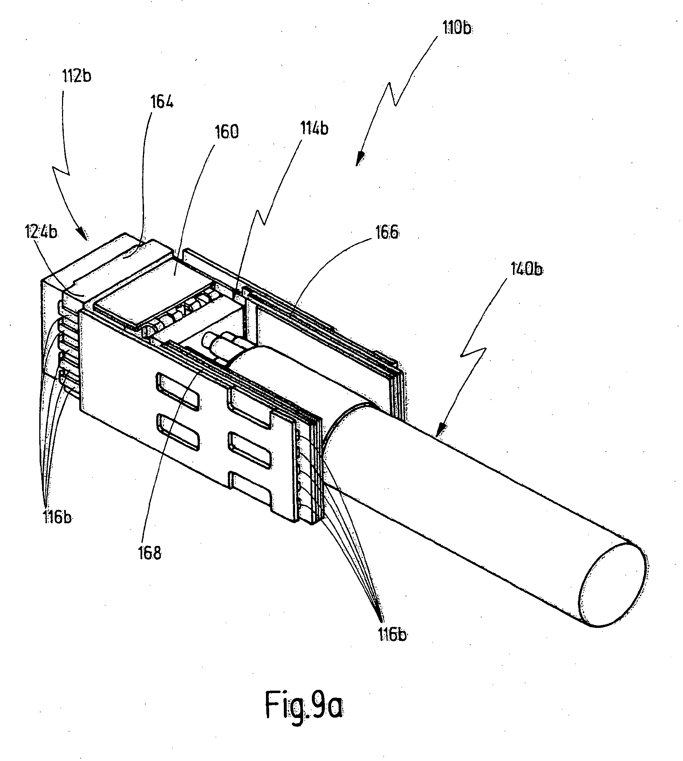

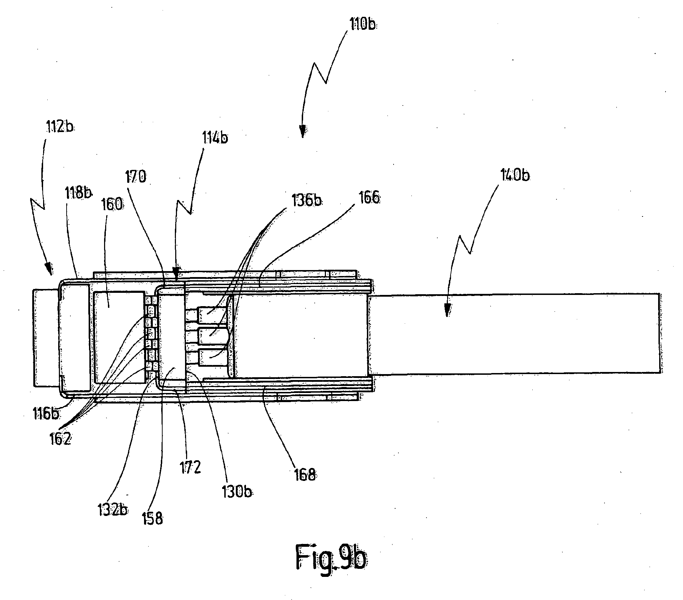

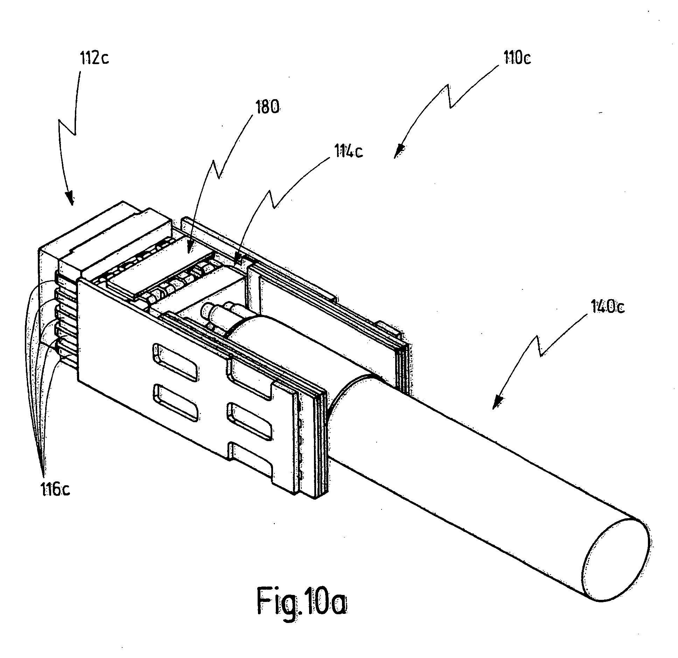

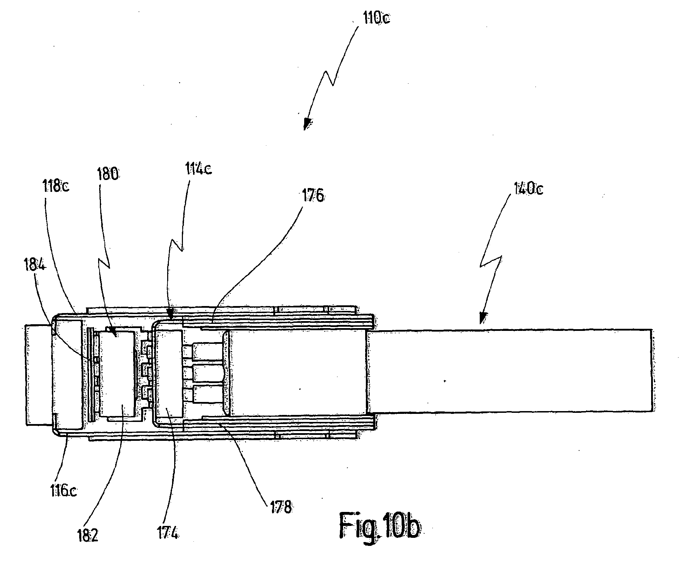

In Fig. 9 und 10 sind zwei weitere Abwandlungen des Bildaufnehmermoduls 110 dargestellt.

Fig. 9a und b) zeigen ein Bildaufnehmermodul 110b mit den Hauptkomponenten Bildsensor 112b, Platine 114b und flexibles mehradriges Kabel 140b.

Die Platine 114b ist im Unterschied zu der Platine 114 oder 114a mehrteilig aufgebaut.

Die Platine 114b weist eine starre Basisplatine 158 auf, deren Funktion in diesem Fall die Kontaktierung von Adern 136b eines mehradrigen flexiblen Kabels 140b ist. Die Adern 136b sind wiederum vorzugsweise durch nicht näher dargestellte Bohrungen von der Unterseite 130b zur Oberseite 132b der Basisplatine 158 durchgeführt und auf der Oberseite 132b mit der Basisplatine 158 kontaktiert.

Die Basisplatine 158 trägt vorzugsweise keine elektronischen Bauelemente. Für die Aufnahme elektronischer Bauelemente weist die Platine 114b vielmehr zumindest ein weiteres Platinenteil 160 auf, das mit der Basisplatine 158 flexibel verbunden ist und zwar über Leiter oder Leiterbahnen 162. Das weitere Platinenteil 160 ist in Form einer dünnen Platte ausgebildet und erstreckt sich senkrecht zu dem Bildsensor 112b in dem Raum zwischen der Basisplatine 158 und dem Bildsensor 112b.

Das Platinenteil 160 erstreckt sich etwa in Verlängerung einer Längsseite 164 des Bildsensors 112a bzw. dessen Basiskörpers 124a, und kann auf seiner Innenseite (nicht dargestellt) ein oder mehrere elektronische Bauelemente tragen, die mit dem Platinenteil 160 elektrisch kontaktiert sind. Es kann auch parallel zum Platinenteil 160 auf der gegenüberliegenden Längsseite ein weiteres solches Platinenteil angeordnet sein.

Mit der Basisplatine 158 sind zwei weitere Platinenteile 166 und 168 flexibel verbunden, und zwar über Leiter 170 bzw. 172.

Die Platinenteile 166 und 168 verlaufen ebenfalls quer zum Bildsensor 112b und sind auf der Seite der Basisplatine 158 angeordnet, auf der auch die Kontaktfinger 116b bzw. 118b verlaufen, die jedoch an der Basisplatine 158 vorbeilaufen und mit den Platinenteilen 166 bzw. 168 kontaktiert sind.

Die Platinenteile 166 und 168 dienen u.a. dazu, das mehradrige Kabel 140b teilweise einzufassen, ohne jedoch die Flexibilität des Kabels 140b zu beeinträchtigen.

Eine Abwandlung des Bildaufnehmermoduls 110b ist in Fig. 10a) und b) in Form eines Bildaufnehmermoduls 110c dargestellt. Teile des Bildaufnehmermoduls 110c, die mit entsprechenden Teilen des Bildaufnehmermoduls 110 gleich oder vergleichbar sind, sind mit den gleichen Bezugszeichen, ergänzt durch den Buchstaben c, versehen.

Nachfolgend werden nur die Unterschiede zu dem Bildaufnehmermodul 110c beschrieben.

Das Bildaufnehmermodul 110c weist eine Platine 114c auf, die wie bei dem vorherigen Ausführungsbeispiel eine Basisplatine 174, zwei von der Basisplatine 174 quer zum Bildsensor 112c nach proximal führende Platinenteile 176, 178 und ein von der Basisplatine 174 nach distal wegführendes und sich quer zum Bildsensor 112 erstreckendes Platinenteil 180 aufweist.

Das Platinenteil 180 weist einen ersten Abschnitt 182 und einen sich daran anschließenden zweiten Abschnitt 184 auf, wobei die Abschnitte 182 und 184 senkrecht zueinander verlaufen und miteinander elektrisch leitend und flexibel verbunden sind, wobei sich der Abschnitt 182 senkrecht zur Basisplatine 174 und der Abschnitt 184 parallel zur Basisplatine und parallel zum Bildsensor 112c erstreckt.

Sowohl mit dem Abschnitt 182 als auch mit dem Abschnitt 184 können elektronische Bauelemente kontaktiert werden.

Bei den Ausführungsbeispielen gemäß Fig. 9 und 10 können die Basisplatine und die dazugehörigen Platinenteile aus einem Platinenrohling gefertigt werden, wobei die Basisplatine gegenüber den weiteren Platinenteilen eine größere Materialstärke aufweist, beispielsweise durch nachträgliches Anfügen eines Trägermaterials an die Basisplatine oder durch Wegnehmen von Trägermaterial von den Platinenteilen.

Bei allen zuvor beschriebenen Ausführungsbeispielen können die Platinen oder Einzelplatinen, wie beispielsweise die Platine 26, 26a-e, 114, 114a-c als Multilayerplatinen ausgebildet sein. Beispielsweise im Falle der Platine 26 kann diese aus mehreren Schichten oder Lagen aufgebaut sein, so dass Leiterbahnen auch im Innern des Platinenkörpers verlaufen.

## Patentansprüche

1. Bildaufnehmermodul, insbesondere für ein Endoskop oder eine Miniaturkamera, mit einem elektronischen Bildsensor (112), der eine Mehrzahl von Kontaktfingern (116, 118) aufweist, die in zumindest einer Reihe angeordnet sind, und mit einer starren Platine (114), mit der die Kontaktfinger (116, 118) elektrisch kontaktiert sind, wobei der Bildsensor (112) und die Platine (114) etwa parallel zueinander angeordnet sind, und wobei die Kontaktfinger (116, 118) sich entlang zumindest einer, sich etwa quer zum Bildsensor (112) erstreckenden Längsseite (120, 122) der Platine erstrecken, ferner mit einem flexiblen mehradrigen Kabel (140), das von der Platine (114) in Richtung von dem Bildsensor (112) wegführt und dessen Adern (136, 138) ebenfalls mit der Platine (114) elektrisch kontaktiert sind, wobei die Adern (136, 138) an Kontaktierstellen (142) an der Platine kontaktiert sind, die näher zum Bildsensor (112) liegen als die vom Bildsensor (112) abgewandte Seite (130) der Platine (114), **dadurch gekennzeichnet, dass** sich die Kontaktierstellen (142) der Adern (136, 138) auf der dem Bildsensor (112) zugewandten Seite der Platine (114) befinden, und die Adern (136, 138) mit ihren Ummantelungen von der dem Bildsensor (112) abgewandten Seite (130) zur dem Bildsensor zugewandten Seite (132) durch die Platine (114) durchgeführt sind.

2. Bildaufnehmermodul nach Anspruch 1, **dadurch gekennzeichnet, dass** die Platine (114) einteilig ausgebildet ist.

3. Bildaufnehmermodul nach Anspruch 2, **dadurch gekennzeichnet, dass** die Platine (114) als Multilayerplatine ausgebildet ist.

4. Bildaufnehmermodul nach einem der Ansprüche 1 bis 3, **dadurch gekennzeichnet, dass** die Platine als Anordnung aus parallel zueinander und parallel zum Bildsensor verlaufenden starren Einzelplatinen mehrteilig ausgebildet ist.

5. Bildaufnehmermodul nach Anspruch 4, **dadurch gekennzeichnet, dass** die Einzelplatinen durch die Kontaktfinger des Bildsensors miteinander verbunden sind.

6. Bildaufnehmermodul nach Anspruch 4 oder 5, **dadurch gekennzeichnet, dass** die Einzelplatinen über einzelne Leiter oder Leiterbahnen miteinander verbunden sind.

7. Bildaufnehmermodul nach einem der Ansprüche 4 bis 6, **dadurch gekennzeichnet, dass** die Einzelplatinen Vertiefungen in Form von Nuten und/oder Sacklochbohrungen zur Aufnahme von elektronischen Bauelementen aufweisen.

8. Bildaufnehmermodul nach einem der Ansprüche 4 bis 6, **dadurch gekennzeichnet, dass** sich die Kontaktierstellen der Adern auf einer dem Bildsensor zugewandten Seite der vom Bildsensor (12c) aus gesehen letzten Einzelplatine befinden.

9. Bildaufnehmermodul nach einem der Ansprüche 4 bis 8, **dadurch gekennzeichnet, dass** zumindest eine der Einzelplatinen (62, 64; 70, 72; 78, 80) als Multilayerplatine ausgebildet ist.

10. Bildaufnehmermodul nach einem der Ansprüche 1 bis 9, **dadurch gekennzeichnet, dass** die Platine von der dem Bildsensor abgewandten Seite zur dem Bildsensor zugewandten Seite Durchkontaktierungen aufweist.

11. Bildaufnehmermodul nach einem der Ansprüche 1 bis 10, **dadurch gekennzeichnet, dass** die Platine (114) von dem Bildsensor (112) beabstandet ist.

12. Bildaufnehmermodul nach Anspruch 11, **dadurch gekennzeichnet, dass** in dem Raum (126) zwischen dem Bildsensor (112) und der Platine (114) zumindest ein elektronisches Bauelement (128) angeordnet ist.

13. Bildaufnehmermodul nach Anspruch 11 oder 12, **dadurch gekennzeichnet, dass** der Raum (126) zwischen der Platine (114) und dem Bildsensor (112) mit einem aushärtbaren elektrisch isolierenden Füllmaterial ausgegossen ist.

14. Bildaufnehmermodul nach einem der Ansprüche 1 bis 13, **dadurch gekennzeichnet, dass** die Kontaktfinger (116, 118) des Bildsensors (112) an zumindest einer Längsseite (120, 122) der Platine (114) kontaktiert sind, an der vertieft angeordnete längliche Kontakte (144, 146) vorhanden sind.

15. Bildaufnehmermodul nach einem der Ansprüche 1 bis 14, **dadurch gekennzeichnet, dass** die Platine (114b; 114c) eine starre Basisplatine (158; 174) aufweist, die zur Kontaktierung des mehradrigen Kabels (140b; 140c) dient, und dass mit der Basisplatine (158; 174) zumindest ein erstes weiteres Platinenteil (160; 180) flexibel verbunden ist, das sich zumindest teilweise etwa senkrecht zur Basisplatine (158; 174) erstreckt.

16. Bildaufnehmermodul nach Anspruch 15, **dadurch gekennzeichnet, dass** das zumindest eine erste weitere Platinenteil (160; 180) zur Befestigung und Kontaktierung zumindest eines elektronischen Bauelements dient.

17. Bildaufnehmermodul nach Anspruch 15 oder 16, **dadurch gekennzeichnet, dass** das zumindest eine erste weitere Platinenteil (180) zumindest einen ersten Abschnitt (182) aufweist, der sich etwa senkrecht von der Basisplatine (174) weg zum Bildsensor (112c) hin erstreckt, und zumindest einen zweiten Abschnitt (184), der sich an den ersten Abschnitt (182) anschließt und etwa parallel zur Basisplatine (174) erstreckt.

18. Bildaufnehmermodul nach einem der Ansprüche 15 bis 17, **dadurch gekennzeichnet, dass** die Basisplatine (158; 174) mit zumindest zwei weiteren zweiten und dritten Platinenteilen (166, 168; 176, 178) flexibel verbunden ist, die von zwei gegenüberliegenden Längsseiten der Basisplatine (158; 174) senkrecht zu dieser wegführen und das mehradrige Kabel (140b; 140c) einfassen.

19. Bildaufnehmermodul nach einem der Ansprüche 15 bis 18, **dadurch gekennzeichnet, dass** die Basisplatine (158; 174) und den zumindest eine erste und ggfls zumindest eine zweite und dritte weitere Platinenteil (160, 166, 168; 176, 178, 180) mit der Basisplatine (158; 174) aus einem Platinenrohling gefertigt sind, wobei die Basisplatine (158; 174) gegenüber der oder den weiteren Platinenteilen (160, 166, 168; 176, 178, 180) eine größere Materialstärke aufweist.

## Claims

1. An image pick-up module, in particular for an endoscope or a miniature camera, comprising an electronic image sensor (112), which has a plurality of contact fingers (116, 118) that are arranged in at least one row, and a rigid circuit board (114) to which the contact fingers (116, 118) are electrically contact-connected, the image sensor (112) and the circuit board (114) being arranged approximately parallel to one another, and the contact fingers (116, 118) extending along at least one longitudinal side (120, 122) of the circuit board, which longitudinal side extends approximately transversely to the image sensor (112), and further comprising a flexible multi-core cable (140) which leads from the circuit board (114) in the direction away from the image sensor (112) and whose cores (136, 138) are likewise electrically contact-connected to the circuit board (114), wherein the cores (136, 138) are contact-connected at contact-connection points (142) on the circuit board which are closer to the image sensor (112) than that side (130) of the circuit board (114) which is remote from the image sensor (112), **characterized in that** the contact-connection points (142) of the cores (136, 138) are situated on the side of the circuit board (114) which faces the image sensor (112), and the cores (136, 138) together with their sheaths are guided through the circuit board (114) from the side (130) which is facing away from the image sensor (112) to the side (132) which faces the image sensor.

2. The image pick-up module of claim 1, **characterized in that** the circuit board (114) is of single-part design.

3. The image pick-up module of claim 2, **characterized in that** the circuit board (114) is in the form of a multilayer circuit board.

4. The image pick-up module of any one of claims 1 through 3, **characterized in that** the circuit board is of multi-part design in the form of an arrangement of rigid individual circuit boards which run parallel to one another and parallel to the image sensor.

5. The image pick-up module of claim 4, **characterized in that** the individual circuit boards are connected to one another by means of the contact fingers of the image sensor.

6. The image pick-up module of claim 4 or 5, **characterized in that** the individual circuit boards are connected to one another by means of individual conductors or conductor tracks.

7. The image pick-up module of any one of claims 4 through 6, **characterized in that** the individual circuit boards have depressions in the form of grooves and/or blind holes for accommodating electronic components.

8. The image pick-up module of any one of claims 4 through 6, **characterized in that** the contact connection points of the cores are situated on a side of the last individual circuit board, as seen from the image sensor (12c), which faces the image sensor.

9. The image pick-up module of any one of claims 4 through 8, **characterized in that** at least one of the individual circuit boards (62, 64; 70, 72; 78, 80) is in the form of a multilayer circuit board.

10. The image pick-up module of any one of claims 1 through 9, **characterized in that** the circuit board has throughplated holes from the side which faces away from the image sensor to the side which faces the image sensor.

11. The image pick-up module of any one of claims 1 through 10, **characterized in that** the circuit board (114) is spaced apart from the image sensor (112).

12. The image pick-up module of claim 11, **characterized in that** at least one electronic component (128) is arranged in the space (126) between the image sensor (112) and the circuit board (114).

13. The image pick-up module of claim 11 or 12, **characterized in that** the space (126) between the circuit board (114) and the image sensor (112) is filled with a curable electrically insulating filling material.

14. The image pick-up module of any one of claims 1 through 13, **characterized in that** the contact fingers (116, 118) of the image sensor (112) are contact-connected on at least one longitudinal side (120, 122) of the circuit board (114) on which elongate contacts (144, 146) which are arranged so as to be recessed are provided.

15. The image pick-up module of any one of claims 1 through 14, **characterized in that** the circuit board (114b; 114c) has a rigid base circuit board (158; 174) which is used to contact-connect the multi-core cable (140b; 140c), and **in that** at least one first further circuit board part (160; 180) which at least partially extends approximately perpendicular to the base circuit board (158; 174) is flexibly connected to the base board (158; 174).

16. The image pick-up module of claim 15, **characterized in that** the the at least one first further circuit board part (160; 180) is used to fasten and contact-connect at least one electronic component.

17. The image pick-up module of claim 15 or 16, **characterized in that** the at least one first further circuit board part (180) has at least one first section (182), which extends in an approximately perpendicular manner away from the base circuit board (174) towards the image sensor (112c), and at least one second section (184) which adjoins the first section (182) and extends approximately parallel to the base circuit board (174).

18. The image pick-up module of any one of claims 15 through 17, **characterized in that** the base circuit board (158; 174) is flexibly connected to at least two further second and third circuit board parts (166, 168; 176, 178) which lead away from two opposite longitudinal sides of the base circuit board (158; 174) perpendicular to the latter and enclose the multi-core cable (140b; 140c).

19. The image pick-up module of any one of claims 15 through 18, **characterized in that** the the base circuit board (158; 174) and the at least one first and optionally at least one second and third further circuit board part (160, 166, 168; 176, 178, 180) are produced, together with the base circuit board (158; 174), from a circuit board blank, the base circuit board (158; 174) having a greater material thickness than the further circuit board part(s) (160, 166, 168; 176, 178, 180).

## Revendications

1. Module imageur, en particulier destiné à un endoscope ou à une caméra miniature, comprenant un capteur d'image (112) électronique, qui présente une pluralité de doigts de contact (116, 118), qui sont disposés en au moins une rangée, et comprenant une platine (114) rigide, avec laquelle les doigts de contact (116, 118) sont mis en contact électrique, sachant que le capteur d'image (112) et la platine (114) sont disposés approximativement de manière parallèle l'un par rapport à l'autre, et sachant que les doigts de contact (116, 118) s'étendent le long d'au moins un côté longitudinal (120, 122) de la platine s'étendant approximativement de manière transversale par rapport au capteur d'image (112), comprenant en outre un câble (140) flexible à plusieurs brins, qui part de la platine (114) en direction du capteur d'image (112) et dont les brins (136, 138) sont de la même manière mis en contact électrique avec la platine (114), sachant que les brins (136, 138) sont mis en contact au niveau de la platine à des emplacements de mise en contact (142) se trouvant davantage à proximité du capteur d'image (112) que le côté (130), opposé au capteur d'image (112), de la platine (114), **caractérisé en ce que** les emplacements de mise en contact (142) des brins (136, 138) se trouvent sur le côté, tourné vers le capteur d'image (112), de la platine (114), et **en ce que** les brins (136, 138) sont amenés à traverser la platine (114), par leurs enveloppes depuis le côté (130) opposé au capteur d'image (112) vers le côté (132) tourné vers le capteur d'image.

2. Module imageur selon la revendication 1, **caractérisé en ce que** la platine (114) est réalisée en une seule partie.

3. Module imageur selon la revendication 2, **caractérisé en ce que** la platine (114) est réalisée sous la forme d'une platine multicouche.

4. Module imageur selon l'une quelconque des revendications 1 à 3, **caractérisé en ce que** la platine est réalisée en plusieurs parties sous la forme d'un ensemble composé de platines individuelles rigides s'étendant de manière parallèle les unes aux autres et de manière parallèle par rapport au capteur d'image.

5. Module imageur selon la revendication 4, **caractérisé en ce que** les platines individuelles sont connectées les unes aux autres par les doigts de contact du capteur d'image.

6. Module imageur selon la revendication 4 ou 5, **caractérisé en ce que** les platines individuelles sont connectées les unes aux autres par l'intermédiaire de conducteurs ou de pistes conductrices individuels.

7. Module imageur selon l'une quelconque des revendications 4 à 6, **caractérisé en ce que** les platines individuelles présentent des renfoncements sous la forme de rainures et/ou de trous borgnes servant à recevoir des composants électroniques.

8. Module imageur selon l'une quelconque des revendications 4 à 6, **caractérisé en ce que** les emplacements de mise en contact des brins se trouvent sur un côté, tourné vers le capteur d'image, de la dernière platine individuelle vu depuis le capteur d'image (12c).

9. Module imageur selon l'une quelconque des revendications 4 à 8, **caractérisé en ce qu'**au moins une des platines individuelles (62, 64 ; 70, 72 ; 78, 80) est réalisée sous la forme d'une platine multicouche.

10. Module imageur selon l'une quelconque des revendications 1 à 9, **caractérisé en ce que** la platine présente des passages d'interconnexion allant du côté opposé au capteur d'image vers le côté tourné vers le capteur d'image.

11. Module imageur selon l'une quelconque des revendications 1 à 10, **caractérisé en ce que** la platine (114) est tenue à distance du capteur d'image (112).

12. Module imageur selon la revendication 11, **caractérisé en ce qu'**au moins un composant électronique (128) est disposé dans l'espace (126) entre le capteur d'image (112) et la platine (114).

13. Module imageur selon la revendication 11 ou 12, **caractérisé en ce que** l'espace (126) entre la platine (114) et le capteur d'image (112) est coulée avec un matériau de remplissage durcissable à isolation électrique.

14. Module imageur selon l'une quelconque des revendications 1 à 13, **caractérisé en ce que** les doigts de contact (116, 118) du capteur d'image (112) sont mis en contact au niveau au moins d'un côté longitudinal (120, 122) de la platine (114), au niveau duquel sont disponibles des contacts (144, 146) allongés disposés en creux.

15. Module imageur selon l'une quelconque des revendications 1 à 14, **caractérisé en ce que** la platine (114b ; 114c) présente une platine de base (158 ; 174) rigide, qui sert à la mise en contact du câble (140b ; 140c) multibrin, et **en ce qu'**au moins une autre première partie de platine (160; 180) est connectée de manière flexible à la platine de base (158 ; 174), laquelle partie de platine s'étend au moins en partie approximativement de manière perpendiculaire par rapport à la platine de base (158 ; 174).

16. Module imageur selon la revendication 15, **caractérisé en ce que** la au moins une première partie de platine (160 ; 180) supplémentaire sert à fixer et à mettre en contact au moins un composant électronique.

17. Module imageur selon la revendication 15 ou 16, **caractérisé en ce que** la au moins une première partie de platine (180) supplémentaire présente au moins une première section (182), qui s'étendant approximativement de manière perpendiculaire en s'éloignant de la platine de base (174) en direction du capteur d'image (112c), et au moins une deuxième section (184), qui se raccorde à la première section (182) et s'étend approximativement de manière parallèle en direction de la platine de base (174).

18. Module imageur selon l'une quelconque des revendications 15 à 17, **caractérisé en ce que** la platine de base (158 ; 174) est connectée de manière flexible à au moins deux deuxième et troisième parties de platine (166, 168 ; 176, 178) supplémentaires, lesquelles partent des deux côtés longitudinaux se faisant face de la platine de base (158 ; 174) de manière perpendiculaire en direction de cette dernière et renferment le câble (140b ; 140c) multibrin.

19. Module imageur selon l'une quelconque des revendications 15 à 18, **caractérisé en ce que** la platine de base (158; 174) et la au moins une première et éventuellement au moins une deuxième et troisième partie de platine (160, 166, 168 ; 176, 178, 180) supplémentaire sont fabriquées avec la platine de base (158 ; 174) à partir d'une ébauche de platine, sachant que la platine de base (158 ; 174) présente, contrairement à la partie de platine ou aux parties de platine (160, 166, 168 ; 176, 178, 180) supplémentaire(s), une épaisseur de matériau plus importante.
